# EUROPÄISCHE PATENTSCHRIFT

(11) **EP 3 011 090 B1**
(45) Veröffentlichungstag und Bekanntmachung des Hinweises auf die Patenterteilung: **15.02.2017**
(21) Anmeldenummer: 14747290.6
(22) Anmeldetag: 13.06.2014
(51) Int. Cl.: D01F 9/00, D01F 2/06, C08B 37/00, C08L 5/00, D04H 1/28, D04H 3/013

(54) **HOCHSAUGFÄHIGE POLYSACCHARIDFASER UND IHRE VERWENDUNG**
HIGHLY ABSORBENT POLYSACCHARIDE FIBER AND USE THEREOF
FIBRES DE POLYSACCHARIDE À FORT POUVOIR ABSORBANT ET LEUR UTILISATION

(30) Priorität: 17.06.2013 AT 4832013
(43) Veröffentlichungstag der Anmeldung: 27.04.2016
(73) Patentinhaber: Lenzing AG, 4860 Lenzing (AT)
(72) Erfinder: RÖDER, Thomas, A-4840 Vöcklabruck (AT); KAINDL, Gernot, A-4860 Lenzing (AT); REDLINGER, Sigrid, A-4860 Lenzing (AT); FIRGO, Heinrich, A-4840 Vöcklabruck (AT); KRONER, Gert, A-4863 Seewalchen (AT)
(74) Vertreter: Hanemann, Otto
(86) Internationale Anmeldenummer: PCT/AT2014/000125
(87) Internationale Veröffentlichungsnummer: WO 2014/201484

(56) Entgegenhaltungen:
- WO-A1-97/04148
- WO-A1-2013/052730
- DE-A1- 3 036 415

## Beschreibung

Die vorliegende Erfindung betrifft eine neuartige, hochsaugfähige Polysaccharidfaser, ihre Herstellung und Eigenschaften und deren Verwendung.

### Stand der Technik

Vliesstoffe, englisch "Nonwovens", sind aus textilen Fasern aufgebaute, poröse Flächengebilde. Hinsichtlich der Längen der eingesetzten Fasern wird zwischen Spinnvliesen aus Endlosfasern, die durch unmittelbares Ablegen der Fasern nach dem Spinnprozess erhalten werden können, und Spinnfaservliesen aus Fasern mit definierter Schnittlänge, unterschieden.

Diese werden entweder auf trockenem Wege, zum Beispiel durch Verpressen von Kardenbändern, wie es bei der Tamponherstellung der Fall ist, oder auf nassem Wege, z. B. ähnlich der Papierherstellung mit anschließender Verfestigung, hergestellt. Neben natürlichen Fasern wie Wolle oder
Baumwolle finden auch Chemiefasern, darunter Polypropylen oder Polyester Verwendung. Im Bereich der absorbierenden Nonwovens-Produkte werden aufgrund ihres äußerst hydrophilen Charakters zu einem überwiegenden Teil cellulosische Fasern eingesetzt. Deren hohe Absorptionskraft beruht auf der Eigenschaft der Cellulose, mit Wassermolekülen starke Wasserstoffbrücken zu bilden. Zudem zeichnen sich diese Fasern durch vollständige biologische Abbaubarkeit aus. Neben Baumwolle und Zellstoff werden vor allem manmade-Cellulosefasern, sogenannte Celluloseregeneratfasern, wie Viskose- oder Lyocellfasern eingesetzt, da diese natürliche Cellulosefasern wie Baumwolle in Bezug auf Reinheit, Weichheit und Saugeigenschaften in weiten Bereichen übertreffen. Viscose- und Modal-Verfahren sollen für die Zwecke der vorliegenden Erfindung auch zusammenfassend als "Xanthogenatverfahren" bezeichnet werden, da in ihnen stets Polysaccharide mit CS2 zu den entsprechenden Xanthogenaten umgesetzt werden. Xanthogenat-Verfahren zur Herstellung von Cellulosefasern sind dem Fachmann seit Jahrzehnten grundsätzlich bekannt.

Beispiele für absorbierende Nonwovens-Produkte umfassen Wisch- und Reinigungstücher, Hygieneartikel wie Tampons oder Damenbinden, sterile Abdecktücher oder Wundpflegeprodukte für medizinische Anwendungen und kosmetische Erzeugnisse wie Reinigungspads oder Erfrischungstücher. Die Anforderungen an diese Erzeugnisse variieren in Abhängigkeit des Verwendungszwecks zum Teil erheblich. Wenngleich gewisse Mindesterfordernisse bestehen, vor allem hinsichtlich Faserdehnung und Schlingenfestigkeit, um ein problemloses Kardieren zu ermöglichen, sind die Ansprüche an die mechanischen Eigenschaften der Fasern weitaus geringer als im textilen Bereich. Wesentliche Aufgaben von absorbierenden Nonwovens betreffen die Aufnahme, den Transport, die Verteilung, die Freisetzung und/oder das Zurückhalten von Flüssigkeiten unter den jeweiligen Gebrauchsbedingungen. Zur Beurteilung dieser Eigenschaften haben sich zahlreiche Testmethoden etabliert, darunter das Wasserrückhaltevermögen nach DIN 53814, die Sinkzeit, das Wasserhaltevermögen, die Saugkapazität und Sauggeschwindigkeit gemäß Demand-Wettability-Test, die Dickenquellung und die Wasserdampfaufnahme. Die wichtigste Anforderung an die eingesetzten Fasern im Bereich absorbierender Nonwovens ist ein hohes Aufnahmevermögen für Wasser bzw. Flüssigkeiten im Allgemeinen, darunter Blut oder Harn. Zu dessen Quantifizierung werden vor allem das Wasserrückhaltevermögen und das Wasserhaltevermögen verwendet.

Das Wasserrückhaltevermögen, auch Quellwert genannt, gibt die Menge des festgehaltenen Wassers nach Benetzung und definiertem Abschleudern bezogen auf das trockene Ausgangsgewicht der Fasern in Prozent wieder. Es wird hauptsächlich durch die übermolekulare Faserstruktur und die Porencharakteristik bestimmt.

Das Wasserhaltevermögen entspricht jener Wassermenge, die von einem Faserbausch nach Untertauchen in Wasser und definiertem Abtropfen festgehalten wird. Es handelt sich vor allem um in den Kapillarräumen zwischen den Fasern festgehaltenes Wasser. Wesentliche Einflussgrößen betreffen den Titer, die Kräuselung, die Querschnittsform und die Ausrüstung der Fasern.

Die aus der Literatur bekannten Verfahren zur Herstellung von Celluloseregeneratfasern mit hohem Absorptionsvermögen lassen sich in drei Gruppen gliedern:
1. Physikalische Beeinflussung der Faserstruktur:
   Die Möglichkeiten zur physikalischen Modifizierung der Faserstruktur sind vielfältig und reichen von der Variation der Zusammensetzung der Spinnlösung und des Spinnbades bis zur Beeinflussung der Extrusion des Fadens und der Verstreckung. Durch besondere Absorptionskraft zeichnen sich Hohlfasern, zusammengefallene Hohlfaserstrukturen oder Fasern mit mehrschenkeligen, sogenannten multilobalen Querschnitten aus. Hohlfasern können beispielsweise durch den Zusatz von Natriumcarbonat zur Viskose hergestellt werden. Bei Kontakt mit dem sauren Spinnbad wird Kohlendioxid freigesetzt, das die Fasern aufbläht und zur Ausbildung der Hohlstruktur führt. Die US4129679 (A) beschreibt nach einem derartigen Prozess hergestellte Fasern. Eine Besonderheit dieses Verfahrens ist, dass die aufgeblähten Fasern in sich zusammenfallen und dadurch mehrschenkelige Querschnitte bilden. Weitere Möglichkeiten, Fasern mit multilobaler Querschnittsform herzustellen, sind das Verspinnen der Celluloselösung durch Spinndüsen, deren Öffnungen drei oder mehr Schenkel, bevorzugt mit einem Längen/Breitenverhältnis der Schenkel von 2:1 oder mehr, aufweisen. Ein derartiges Verfahren wird in der WO8901062 (A1) beschrieben. Fasern mit hohem Kräuselungsgrad verfügen ebenfalls über ausgeprägte hydrophile Eigenschaften. Eine Beeinflussung der Kräuselung von Viskosefasern ist beispielsweise durch Verwendung alternativer, kräuselungsgebender Modifier und/oder niedrige Modifierkonzentrationen, die, wie in EP0049710 (A1) beschrieben, unter Umständen bis auf Null reduziert werden können, in Kombination mit geänderten Viskosezusammensetzungen und Spinnbedingungen möglich. Ein Nachteil dieser querschnittsmodifizierten Fasern ist die deutlich verschlechterte Verarbeitbarkeit in den Weiterverarbeitungsschritten (z.B. Kardierung).
2. Beeinflussung durch Inkorporation absorbierender Substanzen, insbesondere Polymere:
   Durch den Zusatz von hydrophilen Polymeren wie Carboxymethylcellulose (US4289824 (A)), Alginsäure oder deren Salze (AT402828 (B)), Guaran (WO9855673 (A1)) oder Copolymerisaten aus Acryl- und Methacrylsäure zur Celluloselösung kann das Wasseraufnahmevermögen von Celluloseregeneratfasern stark erhöht werden. In der DE2550345 (A1) werden Mischfasern aus einer Matrix aus regenerierter Cellulose mit hohem Fluidhaltevermögen durch in der Matrix dispergiertes N-Vinylamidpolymer beschrieben. Die US3844287 (A) schlägt die Herstellung von hochsaugfähigem Material aus Mischfasern aus einer Grundmasse aus Celluloseregenerat, die ein Polyacrylsäuresalz in gleichmäßiger Verteilung enthält, vor. In beiden Fällen erfolgt die Faserherstellung nach dem Viskoseverfahren.
3. Chemische Veränderung der Celluloseregeneratfasern oder der eingesetzten Zellulose:
   Ziel dieser Verfahren ist, das Absorptionsvermögen durch chemische Umsetzungen, die direkt an den Celluloseregeneratfasern oder der faserbildenden Zellulose vorgenommen werden, zu erhöhen. Beispiele sind die Pfropfcopolymerisation der Cellulose mit Acrylsäure oder die Carboxymethylierung von Viskosefasern im niedrigsubstituierten Bereich. Ein derartiges Verfahren hat beispielsweise die JPH0351366 (A) zum Inhalt.

Aus anwendungstechnischer Sicht ist das Wasserrückhaltevermögen der wichtigste Parameter im Bereich absorbierender Nonwovens, da dieser, im Gegensatz zum Wasserhaltevermögen, den praktischen Gegebenheiten stärker entspricht. So genügt es nicht, dass ein Tampon oder eine Wundauflage Körperflüssigkeit nur aufnimmt. Für die Gebrauchsfähigkeit ist es wesentlich, dass die absorbierte Flüssigkeit auch bei Einwirkung von äußeren Kräften innerhalb des Fasermaterials gehalten wird.

Die beschriebenen physikalischen Fasermodifikationen betreffen im Wesentlichen Oberflächeneigenschaften, beispielsweise die Querschnittsform und die Kräuselung der Fasern, und bewirken daher lediglich eine Erhöhung des Wasseraufnahmevermögens. Das Wasserrückhaltevermögen wird nicht oder kaum beeinflusst.

Durch chemische Modifizierungen und/oder durch Inkorporation absorbierender Substanzen lässt sich das Wasserrückhaltevermögen der Fasern verändern, die Einführung nicht-cellulosischer Gruppen ist aber nicht unproblematisch. So kann unter Umständen die biologische Abbaubarkeit nicht mehr in vollem Umfang gegeben sein. Dies ist beispielsweise bei der Inkorporation von Copolymerisaten aus Acryl- und Methacrylsäure der Fall. Ein weiterer Nachteil besteht in der Gefahr des Überschreitens maximal zulässiger Extrakt- oder Aschegehalte. So bildet sich bei der Veraschung natriumcarboxylatgruppenhaltiger Cellulosefasern immer auch eine gewisse Menge an Natriumcarbonat. Die Einbringung geladener Gruppen erschwert die Einhaltung vorgeschriebener pH-Toleranzbereiche. Natriumcarboxylatgruppenhaltige Nonwovens weisen beispielsweise oftmals einen pH-Wert auf, der deutlich im alkalischen Bereich liegt.

Die US 7,000,000 beschreibt Fasern, die durch Verspinnen einer Lösung von Polysacchariden, die im Wesentlichen aus Hexose-Wiederholungseinheiten bestehen, die über α(1→3)-glycosidische Bindungen verknüpft sind, erhalten werden. Diese Polysaccharide können hergestellt werden, indem eine wässrige Lösung von Saccharose mit Glucosyltransferase (GtfJ), isoliert aus Streptococcus salivarius, in Kontakt gebracht wird (Simpson et al. Microbiology, vol 41, pp 1451-1460 (1995)). "Im Wesentlichen" bedeutet in diesem Zusammenhang, dass innerhalb der Polysaccharidketten vereinzelt Fehlstellen auftreten können, an denen andere Bindungskonfigurationen auftreten. Diese Polysaccharide sollen für die Zwecke der vorliegenden Erfindung als "α(1→3)-Glucan" bezeichnet werden.

Gemäß der US 7,000,000 soll das α(1→3)-Glucan derivatisiert, bevorzugt acetyliert, werden. Das Lösungsmittel ist bevorzugt eine organische Säure, eine organische Halogenverbindung, ein fluorierter Alkohol oder eine Mischung aus solchen Komponenten. Diese Lösungsmittel sind teuer und aufwendig zu regenerieren. Über die Absorptionseigenschaften der auf diese Weise hergestellten Fasern offenbart die US 7,000,000 nichts.

Die WO 97/04148 A1 offenbart ein Verfahren zur Herstellung von modifizierten Viskosefasern durch Mischen einer Lösung von Cellulosecarbamat mit einer Lösung von Cellulosexanthogenat. Dieses Verfahren ist sehr aufwendig und komplex, da hierbei zwei Cellulosederivate kombiniert werden, die jeweils zunächst aus Cellulose hergestellt und später jeweils wieder in reine Cellulose überführt werden müssen, wobei dann zwei Arten von abgespaltenen Nebenprodukten anfallen.

Die DE 30 36 415 A1 offenbart Mischfasern auf Basis regenerierter Cellulose mit anionisch modifizierten Polysacchariden als Mischkomponente, wodurch die Fasern hohe Saugfähigkeit und hohes Flüssigkeitsrückhaltevermögen aufweisen. Die anionisch modifizierten Polysaccharide werden in eine Spinnviskose eingemischt. Hierbei handelt es sich um also um Spezialfasern mit sehr speziellen Eigenschaften und begrenztem Anwendungsgebiet.

Die WO 2013/052730 A1 offenbart Fasern mit α(1→3)-Glucan als faserbildende Substanz, die nach dem sogenannten Aminoxid-Verfahren mit NMMO als Lösungsmittel durch Verspinnen hergestellt wurden. Das Aminoxid-Verfahren ist grundsätzlich anders gestaltet als das Viscose- oder das Modal-Verfahren und erfordert eine völlig andere Produktionsanlage. Eine Viscose-Produktionsanlage kann nicht durch einfache Umbaumaßnahmen auf das Aminoxid-Verfahren umgestellt werden.

Zusammenfassend kann festgehalten werden, dass sich Verfahren betreffend die chemische Modifikation der Cellulose oder der Celluloseregeneratfasern und die Inkorporation von hoch absorptiven Substanzen in die Cellulosematrix nicht durchgesetzt haben. Die Gründe dafür sind vielfältig und liegen beispielsweise daran, dass der Mehraufwand aufgrund zusätzlicher Prozessschritte zu hoch ist, eingesetzte hochabsorbierende Substanzen zu teuer oder aus physiologischer und/oder toxikologischer Sicht abzulehnen sind, die gewünschten Absorptionseigenschaften nicht erzielt oder gewisse mechanische Mindeststandards, beispielsweise bei hohen nötigen Füllgraden, nicht erreicht werden.

### Aufgabe

Die Aufgabe bestand gegenüber diesem Stand der Technik darin, eine Faser sowie ein Verfahren zu deren Herstellung zur Verfügung zu stellen, die keine Querschnitts- und chemische Modifizierung erfordert, und aus physiologischer und/oder toxikologischer Sicht völlig unbedenklich ist, aber trotzdem über ein deutlich erhöhtes Wasserrückhaltevermögen verfügt.

### Beschreibung der Erfindung

Die Lösung der oben beschriebenen Aufgabe besteht in einem Verfahren zur Herstellung einer hochsaugfähigen Polysaccharid-Faser nach einem Xanthogenat-Verfahren, wobei die faserbildende Substanz eine Mischung aus Cellulose und α(1→3)-Glucan enthält. Erfindungsgemäß wird das erreicht, indem der Cellulosexanthogenatlösung eine α(1→3)-Glucan-haltige Natronlaugelösung zugesetzt wird. Die Zugabe dieser Glucan-Lösung kann an verschiedenen Stellen des Prozesses erfolgen. Eine solche Polysaccharid-Faser soll für die Zwecke der vorliegenden Erfindung ebenfalls als Viscose- oder Modal-Faser bezeichnet werden, obwohl sie neben Cellulose noch ein weiteres faserbildendes Polysaccharid, nämlich das α(1→3)-Glucan, enthält..

Für die Zwecke der vorliegenden Erfindung soll der Begriff "Faser" sowohl Stapelfasern mit definierter Schnittlänge als auch Endlosfilamente umfassen. Sämtliche im Folgenden beschriebenen Prinzipien der Erfindung gelten grundsätzlich sowohl für Stapelfasern als auch für Endlosfilamente.

Der Einzelfasertiter der erfindungsgemäßen Fasern kann zwischen 0,1 und 10 dtex betragen. Bevorzugt ist er zwischen 0,5 und 6,5 dtex und besonders bevorzugt zwischen 0,9 und 6,0 dtex. Im Falle von Stapelfasern beträgt die Schnittlänge üblicherweise zwischen 0,5 und 120 mm, bevorzugt zwischen 20 und 70 mm und besonders bevorzugt zwischen 35 und 60 mm. Im Falle von Endlosfilamenten beträgt die Anzahl der Einzelfilamente im Filamentgarn zwischen 50 und 10.000, bevorzugt zwischen 50 und 3.000.

Das α(1→3)-Glucan kann hergestellt werden, indem eine wässrige Lösung von Saccharose mit Glucosyltransferase (GtfJ) isoliert aus Streptococcus salivarius in Kontakt gebracht wird (Simpson et al. Microbiology, vol 41, pp 1451-1460 (1995), US 7,000,000).

In einer bevorzugten Ausführungsform des erfindungsgemäßen Verfahrens besteht das α(1→3)-Glucan zu mindestens 90 % aus Hexose-Einheiten und mindestens 50 % der Hexose-Einheiten sind durch α(1→3)-glycosidische Bindungen verknüpft.

Das Verfahren zur Herstellung der erfindungsgemäßen Faser besteht prinzipiell aus folgenden wesentlichen Schritten:
1.a. Herstellung von Alkalicellulose und deren Xanthogenierung
1.b. Herstellung einer alkalischen Glucanlösung
2. Mischung der beiden Lösungen
3. Ausspinnen der α(1→3) Glucan-haltigen Spinnlösung durch eine Düse in ein schwefelsaures Spinnbad, Verstreckung der Fasern und Nachbehandlung.

Die Gesamtkonzentration der faserbildenden Substanz in der Spinnlösung kann zwischen 4 und 15 Gew.-% betragen, bevorzugt sind 5,5 bis 12 Gew.-%.

Die faserbildende Substanz im erfindungsgemäßen Verfahren kann zwischen 1 und 99 Gew.-% α(1→3)-Glucan enthalten. Besonders bevorzugt ist ein Anteil des α(1→3)-Glucans zwischen 5 und 45 Gew.-%. Unterhalb 5% ist der Effekt des α(1→3) Glucan-Zusatzes für übliche Anwendungen der erfindungsgemäßen Fasern zu gering; oberhalb 45% werden Konkurrenzreaktionen um das CS2 in der Spinnlösungen zu groß und die Spinnbarkeit der Lösung nimmt deutlich ab. Beide Grenzen können jedoch unter besonderen Bedingungen bzw. für besondere Anwendungen der erfindungsgemäßen Fasern überschritten werden; auch Fasern mit einem α(1→3)-Glucan-Anteil.zwischen 1 und 5 Gew.-% bzw. zwischen 45 und 99 Gew.-% sind vom Umfang der vorliegenden Erfindung ausdrücklich mit umfasst.

Der restliche Anteil an der faserbildenden Substanz besteht bevorzugt im Wesentlichen aus Cellulose. "Im Wesentlichen" bedeutet in diesem Zusammenhang, dass geringe Mengen anderer Substanzen enthalten sein können, die vor allem aus dem cellulosischen Rohstoff, im Allgemeinen dem Zellstoff stammen. Solche andere Substanzen sind vor allem Hemicellulose und andere Saccharide, Ligninreste oder Ähnliches. Sie sind auch in handelsüblichen Viskose- und Modalfasern enthalten.

Der Umfang der vorliegenden Erfindung soll jedoch ausdrücklich auch solche Fasern umfassen, die neben den bisher genannten Bestandteilen auch weitere Polysaccharide oder funktionale Additive, wie sie in der Nonwovens- und Textilindustrie allgemein bekannt sind, enthalten.

Der Polymerisationsgrad des im erfindungsgemäßen Verfahren eingesetzten α(1→3) Glucans, ausgedrückt als Gewichtsmittel DP_{w}, kann zwischen 200 und 2000 liegen; bevorzugt sind Werte zwischen 500 und 1000.

Gegenstand der vorliegenden Erfindung ist auch eine hochsaugfähige Polysaccharid-Faser, hergestellt nach einem Xanthogenat-Verfahren, die Cellulose und α(1→3)-Glucan enthält. Die faserbildende Substanz der erfindungsgemäßen Faser enthält zwischen 1 und 99 Gew.-%, bevorzugt zwischen 5 und 45 Gew.-% α(1→3)-Glucan

In einer bevorzugten Ausführungsform besteht das α(1→3)-Glucan der erfindungsgemäßen Polysaccharid-Faser zu mindestens 90 % aus Hexose-Einheiten und mindestens 50 % der Hexose-Einheiten sind durch α(1→3)-glycosidische Bindungen verknüpft.

Überraschenderweise wurde gefunden, dass die erfindungsgemäße Faser ein außerordentlich hohes Wasserrückhaltevermögen von mindestens 90% aufweist. Je nach Zusammensetzung und Herstellungsweise ist das Wasserrückhaltevermögen sogar größer als 100%.

Ebenfalls Gegenstand der vorliegenden Erfindung ist die Verwendung der erfindungsgemäßen Fasern zur Herstellung von verschiedensten trocken- und nass gelegten Papieren, Vliesstoffen, Hygieneartikeln wie Tampons, Slipeinlagen und Windeln und sonstigen Vliesstoffen, insbesondere absorbierenden Nonwovens-Produkten, aber auch von textilen Erzeugnissen wie Garnen, Geweben, Gestricken oder Gewirken.

Im Folgenden wird die Erfindung anhand von Beispielen beschrieben. Die Erfindung ist jedoch ausdrücklich nicht auf diese Beispiele beschränkt, sondern umfasst auch alle anderen Ausführungsformen, die auf dem gleichen erfinderischen Konzept beruhen.

### Beispiele

Der Polymerisationsgrad der α(1→3)-Glucane wurde mittels GPC in DMAc/LiCl ermittelt. Im Folgenden wird stets das Gewichtsmittel des Polymerisationsgrades (DP_{w}) angegeben.

### Beispiel 1:

Eine wässrige Viskosexanthogenat-Lösung, enthaltend 29,8 Gew.-% Cellulose, 14,9 Gew.-% NaOH und 8 Gew.-% Schwefel, wurde in einem Löseaggregat mit einer Löselauge 1 enthaltend 4,5 Gew.-% NaOH und danach mit einer Löselauge 2 enthaltend 9 Gew.-% α(1→3)-Glucan und 4,5 Gew.-% NaOH und abschließend mit Wasser umgesetzt. Die so hergestellte Viskose enthielt 8,90 Gew.-% faserbildendes Material, 5,20 Gew.-% NaOH und 2,4 Gew.-% Schwefel (für 100% Cellulose als faserbildendes Material), mit einem Reifeindex von 14 Hottenroth und einer Kugelfallviskosität von 80 Sekunden (bestimmt gemäß dem Zellcheming-Merkblatt III/5/E). Es wurden Viskoselösungen mit 10 und 25% α(1→3)-Glucan hergestellt. Die Glucan-Mengen beziehen sich auf den Anteil des α(1→3)-Glucans an der faserbildenden Substanz. Diese Viskosen enthalten 2,2 Gew.-% Schwefel (10% Glucan und 90% Cellulose als faserbildendes Material) bzw. 1,8 Gew.-% Schwefel (25% Glucan und 75% Cellulose als faserbildendes Material). Die Lösung wurde mittels einer Spinndüse in ein Regenerierbad, enthaltend 100 g/l Schwefelsäure, 330 g/l Natriumsulfat und 15 g/l Zinksulfat, gesponnen. Die Spinndüse hatte 1053 Löcher mit 50µm Durchmesser. Der Viskosespinnlösung wurden 0,5 Gew.-% eines stickstoffhaltigen Hilfsmittels zugesetzt. Zur Erzielung einer entsprechenden Faserfestigkeit erfolgt eine Verstreckung im Zweitbad (92 C, 15 g/l H2S04) von ca. 75%. Die Abzugsgeschwindigkeit beträgt 50 m/min.

In einem Vergleichsbeispiel 1 wurde die Viskose aus Beispiel 1 ohne Zusatz der Glucan-NaOH-Lösung, aber unter ansonsten gleichen Bedingungen wie in Beispiel 1 zu Fasern versponnen.

Die Eigenschaften der erhaltenen Fasern sind in der Tabelle 1 angegeben:

### Beispiel 2:

Eine Viskose, enthaltend 8,70 Gew.-% Cellulose, 5,20 Gew.-% NaOH und 2,3 Gew.-% Schwefel, mit einem Reifeindex von 15 Höttenroth und einer Kugelfallviskosität von 75 Sekunden (bestimmt gemäß dem Zellcheming-Merkblatt III/5/E) wurde mittels einer Spinndüse in ein Regenerierbad, enthaltend 100 g/l Schwefelsäure, 310 g/l Natriumsulfat und 15 g/l Zinksulfat, gesponnen. Die Spinndüse hatte 1053 Löcher mit 50µm Durchmesser. Der Viskosespinnlösung wurden 0,5 Gew.-% eines stickstoffhaltigen Hilfsmittels zugesetzt. Zur Erzielung einer entsprechenden Faserfestigkeit erfolgt eine Verstreckung im Zweitbad (92 C, 15 g/l H2S04) von ca. 75%. Die Abzugsgeschwindigkeit beträgt 50 m/min.

Der Viskoselösung wurden vor der Spinndüse mithilfe einer zwangsfördernden Pumpe entsprechende Mengen einer wässrigen α(1→3)-Glucan-NaOH-lösung (5 Gew.-% NaOH, 8 Gew.-% α(1→3)-Glucan) zugesetzt, so dass Fasern mit 10, 15 und 30% Glucan hergestellt werden konnten. Diese Glucan-Mengen beziehen sich auf den Anteil des α(1→3)-Glucans an der gesamten faserbildenden Substanz der Polysaccharidfasern.

In einem Vergleichsbeispiel 2 wurde die Viskose aus Beispiel 2 ohne Zusatz der Glucan-NaOH-Lösung, aber unter ansonsten gleichen Bedingungen wie in Beispiel 2 zu Fasern versponnen.

Die Eigenschaften der erhabenen Fasern sind in der Tabelle 1 angegeben:

**Tabelle 1**

| Beispiel | Zusatz | Glucan-Menge % | Titer dtex | FFk cN/tex | FDk % | WRV % |
|---|---|---|---|---|---|---|
| Vergleichs-Beispiel 1 | ohne | - | 1,7 | 27,4 | 16,2 | 86 |
| 1a | Glucan DP_{w}800 | 10 | 1,7 | 27,4 | 16,5 | 94 |
| 1b | Glucan DP_{w}800 | 20 | 1,7 | 24,7 | 14,6 | 107 |
| Vergleichs-Beispiel 2 | ohne | - | 1,3 | 29,6 | 15,8 | 87 |
| 2a | Glucan DP_{w} 1000 | 10 | 1,3 | 28,6 | 17,9 | 95 |
| 2b | Glucan DP_{w} 1000 | 15 | 1,3 | 26,1 | 18,1 | 116 |
| 2c | Glucan DP_{w} 1000 | 25 | 1,3 | 23,6 | 19,4 | 124 |

| | | | | | | |
|---|---|---|---|---|---|---|
| FFk Faserfestigkeit konditioniert FDk Faserdehnung konditioniert WRV Wasserückhaltevermögen | | | | | | |

## Patentansprüche

1. Verfahren zur Herstellung einer hochsaugfähigen Polysaccharid-Faser nach einem Xanthogenat-Verfahren, **dadurch gekennzeichnet, dass** die faserbildende Substanz eine Mischung aus Cellulose und α(1→3)-Glucan enthält.

2. Verfahren nach Anspruch 1, wobei die faserbildende Substanz zwischen 1 und 99 Gew.-%, bevorzugt zwischen 5 und 45 Gew.-% α(1→3)-Glucan enthält.

3. Verfahren nach Anspruch 1, wobei das Verfahren ein Viscoseverfahren ist.

4. Verfahren nach Anspruch 1, wobei das α(1→3)-Glucan zu mindestens 90 % aus Hexose-Einheiten besteht und mindestens 50 % der Hexose-Einheiten durch α(1→3)-glycosidische Bindungen verknüpft sind.

5. Verfahren gemäß den vorhergehenden Ansprüchen, wobei die Faser eine Stapelfaser oder ein Endlosfilament ist.

6. Hochsaugfähige Polysaccharid-Faser, hergestellt nach einem Xanthogenat-Verfahren, **dadurch gekennzeichnet, dass** sie als faserbildende Substanz Cellulose und α(1→3)-Glucan enthält.

7. Faser nach Anspruch 6, wobei die faserbildende Substanz zwischen 1 und 99 Gew.-%, bevorzugt zwischen 5 und 45 Gew.-% α(1→3)-Glucan enthält.

8. Faser nach Anspruch 6, wobei das α(1→3)-Glucan zu mindestens 90 % aus Hexose-Einheiten besteht und mindestens 50 % der Hexose-Einheiten durch α(1→3)-glycosidische Bindungen verknüpft sind.

9. Faser gemäß Anspruch 6, wobei die Faser ein Wasserrückhaltevermögen von mindestens 90% aufweist, bevorzugt größer 100%.

10. Faser gemäß Anspruch 6, wobei die Faser eine Stapelfaser oder ein Endlosfilament ist.

11. Verwendung der Faser nach Anspruch 6 zur Herstellung von Vliesstoffen, Hygieneartikeln, insbesondere Tampons, Slipeinlagen und Windeln und sonstigen, absorbierenden Nonwovens-Produkten und Papieren.

12. Verwendung der Faser nach Anspruch 6 zur Herstellung von textilen Erzeugnissen wie Garnen, Geweben, Gestricken oder Gewirken.

## Claims

1. A method for the production of a highly absorbent polysaccharide fiber by using a xanthogenate process, **characterized in that** the fiber-forming substance contains a mixture of cellulose and α(1→3)-glucan.

2. The method as claimed in claim 1, wherein the fiber-forming substance contains between 1 and 99% by weight, preferably between 5 and 45% by weight, of α(1→3)-glucan.

3. The method as claimed in claim 1, wherein the method is a viscose process.

4. The method as claimed in claim 1, wherein at least 90% of said α(1→3)-glucan are hexose units and at least 50% of the hexose units are linked via α(1→3)-glycosidic bonds.

5. The method as claimed in any of the preceding claims, wherein the fiber is a staple fiber or a continuous filament.

6. A highly absorbent polysaccharide fiber produced by using a xanthogenate process, **characterized in that** it contains cellulose and α(1→3)-glucan as the fiber-forming substance.

7. The fiber as claimed in claim 6, wherein the fiber-forming substance contains between 1 and 99% by weight, preferably between 5 and 45% by weight, of α(1→3)-glucan.

8. The fiber as claimed in claim 6, wherein at least 90% of said α(1→3)-glucan are hexose units and at least 50% of the hexose units are linked via α(1→3)-glycosidic bonds.

9. The fiber as claimed in claim 6, wherein the fiber has a water retention capacity of at least 90%, preferably greater than 100%.

10. The fiber as claimed in claim 6, wherein the fiber is a staple fiber or a continuous filament.

11. A use of the fiber as claimed in claim 6 for the production of nonwovens, hygiene articles, particularly tampons, panty liners, and diapers, and of other, absorbent nonwoven products and papers.

12. A use of the fiber as claimed in claim 6 for the production of textile products such as yarns, woven fabrics, or knitted fabrics.

## Revendications

1. Procédé de fabrication d'une fibre de polysaccharide hautement absorbante selon un procédé xanthate de cellulose, **caractérisé en ce que** la substance fibrogène contient un mélange de cellulose et de α(1→3)-glucane.

2. Procédé selon la revendication 1, la substance fibrogène contenant entre 1 et 99 % en masse, de préférence entre 5 et 45 % en masse de α(1→3)-glucane.

3. Procédé selon la revendication 1, le procédé étant un procédé viscose.

4. Procédé selon la revendication 1, le α(1→3)-glucane étant constitué au moins à 90 % d'unités d'hexose, et 50 % des unités d'hexose étant liées par des liaisons glycosidiques en α(1→3).

5. Procédé selon les revendications précédentes, la fibre étant une fibre discontinue ou un filament continu.

6. Fibre de polysaccharide hautement absorbante fabriquée selon un procédé xanthate de cellulose, **caractérisée en ce qu'**elle contient comme substance fibrogène de la cellulose et du α(1→3)-glucane.

7. Fibre selon la revendication 6, la substance fibrogène contenant entre 1 et 99 % en masse, de préférence entre 5 et 45 % en masse de α(1→3)-glucane.

8. Fibre selon la revendication 6, le α(1→3)-glucane étant constitué au moins à 90 % d'unités d'hexose, et 50 % des unités d'hexose étant liées par des liaisons glycosidiques en α(1→3).

9. Fibre selon la revendication 6, la fibre présentant une capacité de rétention d'eau d'au moins 90 %, de préférence supérieure à 100 %.

10. Fibre selon la revendication 6, la fibre étant une fibre discontinue ou un filament continu.

11. Utilisation de la fibre selon la revendication 6 pour la fabrication de non-tissés, d'articles d'hygiène, en particulier de tampons, de protège-slip, de couches et autres produits non-tissés et papiers absorbants.

12. Utilisation de la fibre selon la revendication 6 pour la fabrication de produits textiles comme des fils, tissus ou tricots.
